Europäisches Patentamt

European Patent Office

Office européen des brevets

(11) Veröffentlichungsnummer: **0 286 054**
**A1**

# EUROPÄISCHE PATENTANMELDUNG

(21) Anmeldenummer: 88105407.6

(22) Anmeldetag: 05.04.88

(51) Int. Cl.⁴: **A61B 5/10**

(30) Priorität: 08.04.87 DE 3711923
08.09.87 DE 3730038

(43) Veröffentlichungstag der Anmeldung:
**12.10.88 Patentblatt 88/41**

(84) Benannte Vertragsstaaten:
**FR GB IT SE**

(71) Anmelder: **AMOENA**
**Medizin-Orthopädie-Technik GmbH**
**Kapellenweg 36**
**D-8201 Raubling(DE)**

(72) Erfinder: **Seichert, Nikola, Dr. rer. nat.**
**Galgenbachweg 17a**
**D-8056 Neufahrn(DE)**
Erfinder: **Leyerer, Roland, Dipl.-Ing.**
**Königseestrasse 53**
**D-8208 Kolbermoor(DE)**

(74) Vertreter: **Gossel, Hans K., Dipl.-Ing. et al**
**Rechtsanwälte E. Lorenz - B. Seidler**
**Dipl.-Ing. H. K. Gossel Dr. i. Philipps Dr. P.B.**
**Schäuble Dr. S. Jackermeier - Dipl.-Ing. A.**
**Zinnecker**
**Widenmayerstrasse 23 D-8000 München**
**22(DE)**

(54) **Druckbelastungs-Kontrollgerät.**

(57) Nach Verletzungen der unteren Extremität ist es erforderlich, die von dem Arzt empfohlenen Belastungsstufen einzuhalten. Ein Kontrollgerät für die Druckbelastung der unteren Extremität eines Patienten weist daher in der Sohle eines Schuhs im Fersen-und/oder Ballenbereich ein oder mehrere Druckaufnehmer auf, die dem auf diese wirkenden Druck proportionale elektrische Signale erzeugen. Die Signale werden einer einer elektronischen Auswerteinrichtung zugeführt, die bei Überschreiten eines einstellbaren Druckwertes ein Signal an eine Warneinrichtung abgibt.

Fig. 1

## Druckbelastungs-Kontrollgerät

Die Erfindung betrifft ein Druckbelastungs-Kontrollgerät für Patienten, die nach einem operativen Eingriff an der unteren Extremität stufenweise im einwöchigen Rhytmus ihr operiertes Bein kontrolliert mehrbelasten sollen.

Indikationsbeispiele für die stufenweise Belastung eines Beines sind in erster Linie Umstellungsosteomien, Pfannendachplastiken, Hüftgelenksendoprothesen, Osteosynthese bei Trümmerfrakturen des Femurs etc.. Ein typisches Vorgehen bei diesen Indikationen ist zunächst eine vierwöchige Immobilisierung, an die eine 6-wöchige Belastungsphase mit anfänglich 10 kp Belastung des Beines und nachfolgender stufenweiser wöchentlicher Erhöhung der Belastung um 10 kp anschließt.

Die definierte Einhaltung der empfohlenen Belastung ist schwierig: Das übliche Vorgehen beschränkt sich auf das subjektive Gefühl des Patienten nach kurzzeitigem Üben auf einer Personenwaage. Hierbei dürften sich, je nach Charakter, extreme Schonung und Überbelastung die Waage halten; die korrekte dosierte, stufenweise erhöhte Belastung stellt eher eine Ausnahme dar.

Eine dosierte Belastung der rekonstruierten Strukturen während der Mobilisierung ist speziell für den knöchernen Wiederaufbau von Vorteil, da der Druck hierbei als lokaler Stimulans wirkt. Andererseits birgt eine vorzeitige Überbelastung die Gefahr von Heilungsverzögerung und, im Extremfall, irreversiblen Folgeschäden in sich. Für einen optimalen Heilungsprozeß ist daher eine definierte, stufenweise erhöhte Druckbelastung anzustreben.

Aufgabe der Erfindung ist es daher, durch einen permanenten akustischen Feedback des an der Fußsohle wirkenden Druckes die Einhaltung der vom Arzt empfohlenen Belastungsstufen zu ermöglichen.

Erfindungsgemäß wird diese Aufgabe dadurch gelöst, daß in die Sohle eines geeigneten Schuhes (Sportschuh, Straßenschuh etc.) im Fersen-und/oder Ballenbereich ein oder mehrere Druckaufnehmer angeordnet sind, die dem auf die wirkenden Druck proportionale elektrische Signale erzeugen, und daß die Signale einer elektronischen Auswerteinheit zugeführt werden, die bei Überschreiten eines einstellbaren Durckwertes ein Signal an eine Warneinrichtung abgibt. Mit der erfindungsgemäßen Einrichtung, in einen Schuh eingebaut, kann der Patient normale Gehversuche unternehmen. Sobald die Warneinrichtung ein Signal abgibt, erhöht er die Belastung eines betreffenden Beines nicht mehr und stützt sich beispielsweise auf einer Krücke ab.

Mit der erfindungsgemäßen Vorrichtung ist der Patient nicht mehr allein seinem subjectiven Empfinden ausgeliefert, er wird praktisch bei jedem Schritt in seinem Belastungsverhalten kontrolliert und erfährt damit einen entsprechenden Lerneffekt.

Zweckmäßigerweise addiert die Steuereinrichtung die von den in den Fersen-und Ballenbereichen angeordneten Druckaufnehmern erzeugten Signale und erzeugt bei Überschreiten eines eingestellten Gesamtdruckes ein Warnsignal. Die Warneinrichtung kann dabei eine einen Piepton abgebende Einrichtung oder eine Warnlampe sein.

Ein Ausführungsbeispiel wird nachstehend anhand der Zeichnung näher erläutert. In dieser zeigt

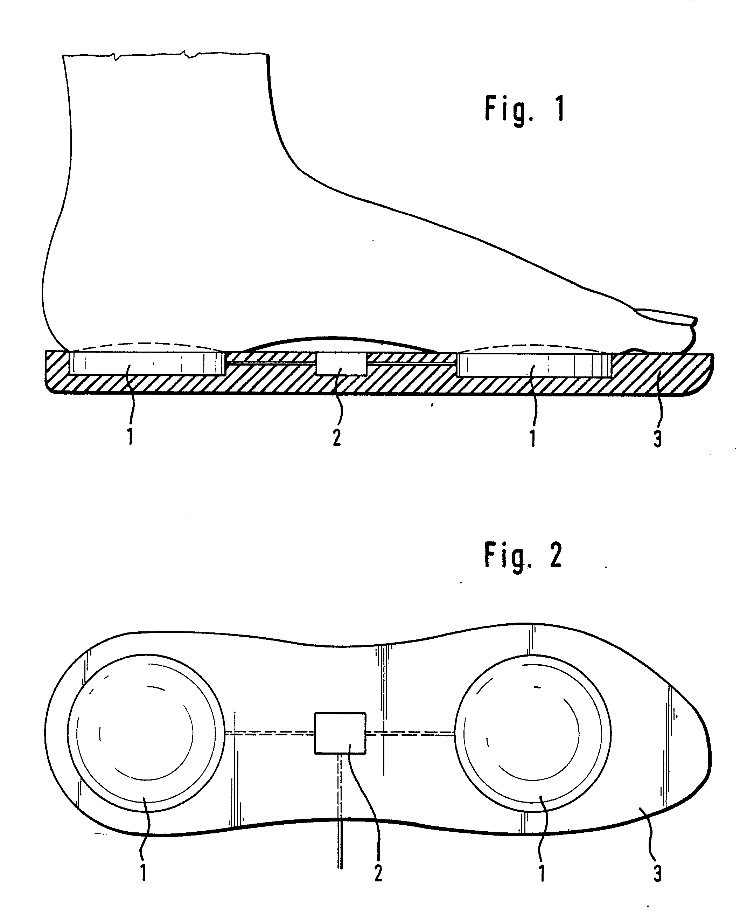

Fig.1 eine Seitenansicht einer Schuhsohle, bei der der besseren Übersichtlichkeit halber das Oberleder fortgelassen ist, und

Fig.2 eine Draufsicht auf die Schuhsohle nach Fig.1

Die Schuhsohle 3 ist in ihrem Fersenbereich un in ihrem Ballenbereich mit kreisrunden Ausnehmungen versehen, in die jeweils in Kunststoff, vorzugsweise Silikonkautschuk, eingelassene oder eingefüllte Drucksensoren 1 eingelassen sind. Die in den Ausnehmungen der Schuhsohle gehalterten Drucksensoren sind durch Kabel mit einem ebenfalls in die Schuhsohle eingelassenen Verbindungsstück 2 verbunden, von dem ein Kabel zu dem elektronischen Auswertesystem führt, das mit einer Warneinrichtung, vorzugsweise einem akustischen Signalgeber, verbunden ist.

Die Steuereinrichtung ist mit einer Stelleinrichtung versehen, durch die sich durch Umlegen von Schaltern verschiedene Druckstufen, beispielsweise von 10-60 kp, einstellen lassen.

## Ansprüche

1. Schuh, **dadurch gekennzeichnet,** daß in der Schuhsohle im Fersen-und/oder Ballenbereich ein oder mehrere Druckaufnehmer angeordnet sind, die dem auf diese wirkenden Druck proportionale elektrische Signale erzeugen, und daß die Signale einer elektronischen Auswerte-und/oder Steuereinrichtung zugeführt werden, die bei Überschreiten eines einstellbaren Druckwertes ein Signal an eine Warneinrichtung abgibt.

2. Schuh nach Anspruch 1, dadurch gekennzeichnet, daß die Steuereinrichtung die von den in den Fersen-und Ballenbereichen angeordneten Druckaufnemern erzeugten Signale addiert und bei Überschreiten eines eingestellten Gesamtdruckes ein Warnsignal erzeugt.

3. Schuh nach Anspruch 1 oder 2, dadurch gekennzeichnet, daß die Warneinrichtung eine einen Piepton abgebende Einrichtung oder eine Warnlampe ist.

# Fig. 1

1    2    1    3

# Fig. 2

1    2    1    3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | MEDICAL AND BIOLOGICAL ENGINEERING, Band 12, Nr. 3, Mai 1974, Seiten 318-321, Stevenage, GB; D. ENDICOTT et al.: "Leg load warning system for the orthopaedically handicapped" * Insgesamt * | 1,3 | A 61 B 5/10 |
| X | FR-A-2 308 923 (THOMSON-CSF) * Figuren 1-3; Seite 1, Zeile 27 - Seite 2, Zeile 24 * | 1 | |
| A | | 3 | |
| A | MEDICAL & BIOLOGICAL ENGINEERING & COMPUTING, Band 16, Nr. 5, September 1978, Seiten 500-506, IFMBE, Stevenage, GB; S. MIYAZAKI et al.: "Limb-load alarm device for partial-weight-bearing walking exercise" * Zusammenfassung; Seiten 501-503, Abschnitt "Device description"; Figuren 1-3 * | 1-3 | |
| A | US-A-3 791 375 (PFEIFFER) * Figuren 1-3; Spalte 2, Zeile 58 - Spalte 4, Zeile 33; Spalte 6, Zeilen 34-54 * | 1-3 | RECHERCHIERTE SACHGEBIETE (Int. Cl.4) A 61 B |
| A | US-A-3 702 999 (GRADISAR) * Figuren 1-3; Spalte 2, Zeile 1 - Spalte 4, Zeile 19 * | 1,3 | |

Der vorliegende Recherchenbericht wurde für alle Patentansprüche erstellt

| Recherchenort | Abschlußdatum der Recherche | Prüfer |
|---|---|---|
| DEN HAAG | 15-07-1988 | CHEN A.H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)